# EUROPEAN PATENT APPLICATION

(11) **EP 1 790 361 A1**
(43) Date of publication of application: **30.05.2007**
(21) Application number: 05026007.4
(22) Date of filing: 29.11.2005
(51) Int. Cl.: A61L 9/22, H01T 23/00

(54) **Ion generator**

(71) Applicant: Samsung Electronics Co., Ltd., Suwon-si, Gyeonggi-Do (KR)
(72) Inventor: Park, Rae Eun, Bundang-Gu,Seongnam-Si,Gyeonggi-Do (KR); Kwon, Jun Hyoun, Gaepo-Dong, Gangnam-Gu, Seoul (KR)
(74) Representative: Grünecker, Kinkeldey, Stockmair & Schwanhäusser Anwaltssozietät

(57) **Abstract**

An ion generator, which generates cations and anions respectively through cation and anion generating units independently installed so as to prevent the extermination of the cations and anions due to reaction therebetween just after the generation of the cations and anions, thereby being capable of generating a large quantity of the cations and anions. The ion generator for sterilization includes a rectifying device and first and second ion generating units The rectifying device rectifies a half wave of the alternating current power into a half-wave alternating current power having a positive polarity (+) and a half-wave alternating current power having a negative polarity (-). The first ion generating unit is operated by the half-wave alternating current power having the positive polarity (+), and generates cations. The second ion generating unit is operated by the half-wave alternating current power having the negative polarity (-), and generates anions.

## Description

### BACKGROUND OF THE INVENTION

### 1. Field of the Invention

The present invention relates to an ion generator, and more particularly to a sterilizing apparatus for eliminating bacteria in the air by generating cations and anions.

### 2. Description of the Related Art

Generally, a conventional air purification apparatus comprises a filter installed in a housing for filtering out various impurities, an air blast fan for exhausting indoor air, which is introduced into the housing and passes through the filter, to the outside of the housing, and an anion generator for generating anions.

When the air blast fan of the air purification apparatus is operated, the indoor air is purified through the filter, and the purified air and the anions generated from the anion generator are exhausted to an indoor space. The sterilization using the filter and the anions of the above conventional air purification apparatus having the anion generator is limited. Thus, an ion generator, which generates cations and anions for sterilization, has been developed. For example, Japanese Patent Laid-open No. 2003-123940 discloses an ion generator for generating cations and anions.

The conventional ion generator applies an alternating current (AC) voltage to a discharge electrode and an induction electrode, generates cations and anions alternately, and supplied the cations and anions to an indoor space. Here, the cations are hydrogen ions (H⁺) and the anions are superoxide anions (O₂⁻). When the hydrogen ions (H⁺) and the superoxide anions (O₂⁻) are supplied to the indoor space, they form hydroxide radicals (OH) or hydrogen peroxide (H₂O₂) and the hydroxide radicals (OH) or hydrogen peroxide (H₂O₂) is attached to bacteria and oxidizes the bacteria, thereby removing the bacteria.

In case that the hydrogen ions (H⁺) and the superoxide anions (O₂⁻), which have negative health effects, generated from the above-described conventional ion generator are exhausted directly to the indoor space and inhaled by users, the hydrogen ions (H⁺) and the superoxide anions (O₂⁻) may damage a user's health.

Further, since the ion generator generates cations and anions alternately, the cations and the anions are reacted with each other and are then destroyed before they can cause sterilization. Particularly, the ion generator, which generates cations and anions alternately, cannot generate a sufficient quantity of the cations and anions for sterilization in a short time. That is, the above conventional ion generator has a small ion-generating capacity.

### SUMMARY OF THE INVENTION

Therefore, an aspect of the invention is to provide an ion generator, which generates cations and anions respectively through cation and anion generating units independently installed so as to prevent the extermination of the cations and anions due to early reaction therebetween, thereby being capable of generating a large quantity of the cations and anions.

In accordance with one aspect, the present invention provides an ion generator for sterilization comprising: a power supply unit for supplying alternating current power; a rectifying device for rectifying a half wave of the alternating current power, supplied from the power supply unit, into a half-wave alternating current power having a positive polarity (+) and a half-wave alternating current power having a negative polarity (-); a first ion generating unit operated by the half-wave alternating current power having the positive polarity (+) for generating cations; and a second ion generating unit operated by the half-wave alternating current power having the negative polarity (-) for generating anions.

In accordance with another aspect, the present invention provides an ion generator comprising: a power supply unit for supplying alternating current power; a rectifying device for rectifying a half wave of the alternating current power, supplied from the power supply unit, into a half-wave alternating current power having a positive polarity (+) and a half-wave alternating current power having a negative polarity (-); a first amplifying device for amplifying the half-wave alternating current power having the positive polarity (+) amplified by the amplifying device and supplying the amplified half-wave alternating current power having the positive polarity (+) to the first ion generating unit; a first ion generating unit for generating cations by receiving the half-wave alternating current power having the positive polarity (+) amplified by the amplifying device; a second amplifying device for amplifying the half-wave alternating current power having the negative polarity (-) amplified by the amplifying device and supplying the amplified half-wave alternating current power having the negative polarity (-) to the second ion generating unit; a second ion generating unit for generating anions by receiving the half-wave alternating current power having the negative polarity (-) amplified by the amplifying device; and a controller for controlling amplification factors of the first and second amplifying devices to control ion-generating capacities of the first and second ion generating units.

### BRIEF DESCRIPTION OF THE DRAWINGS

The above and/or other aspects of the present invention will become apparent and more readily appreciated from the following description of the exemplary embodiments, taken in conjunction with the accompanying drawings in which:
FIG. 1 is a block diagram illustrating the configuration of an ion generator in accordance with an exemplary embodiment of the present invention;
FIG. 2A is a schematic view illustrating a cation generating unit of the ion generator of FIG. 1 in accordance with an exemplary embodiment of the present invention;
FIG. 2B is a schematic view illustrating an anion generating unit of the ion generator of FIG. 1 in accordance with an exemplary embodiment of the present invention; and
FIG. 3 is a schematic view illustrating a cation generating unit and an anion generating unit of the ion generator of FIG. 1 in accordance with another exemplary embodiment of the present invention.

### DETAILED DESCRIPTION OF THE EXEMPLARY EMBODIMENTS

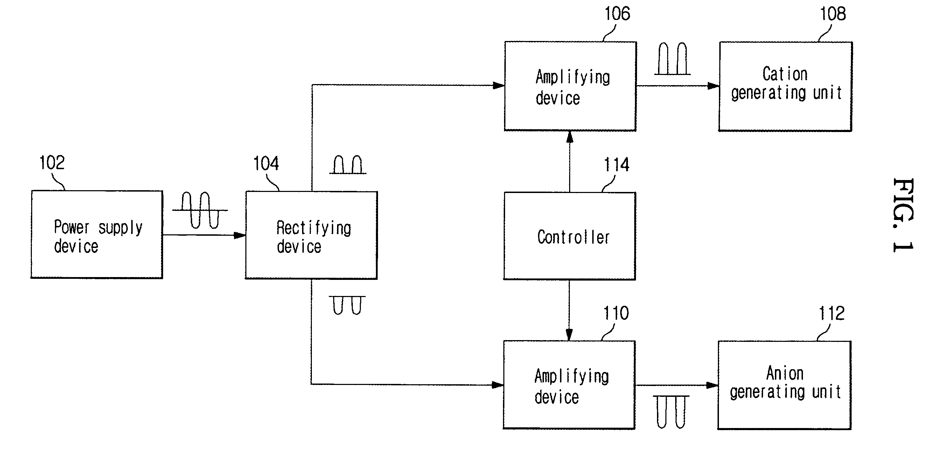

Reference will now be made in detail to the exemplary embodiments of the present invention, examples of which is illustrated in the accompanying drawings, wherein like reference numerals refer to like elements throughout. The exemplary embodiments are described below to explain the present invention by referring to FIGS. 1 to 3.

FIG. 1 is a block diagram illustrating the configuration of an ion generator of according to an exemplary embodiment of the present invention. As shown in FIG. 1, the ion generator comprises two ion generating units, i.e., a cation generating unit 108 for generating cations and an anion generating unit 112 for generating anions, which are operated by half-wave alternating current power rectified by a rectifying device 104.

The rectifying device 104 rectifies a half wave of alternating current power, which is supplied through a power supply device 102, thereby generating a half-wave alternating current power having a positive polarity (+) and a half-wave alternating current power having a negative polarity (-). A first amplifying device 106 amplifies the half-wave alternating current power having the positive polarity (+) generated from the rectifying device 104, and supplies the amplified half-wave alternating current power having the positive polarity (+) to the cation generating unit 108. The cation generating unit 108 is operated by the amplified half-wave alternating current power having the positive polarity (+)to generate cations. A second amplifying device 110 amplifies the half-wave alternating current power having the negative polarity (-) generated from the rectifying device 104, and then supplies the amplified half-wave alternating current power having the negative polarity (-) to the anion generating unit 112. The anion generating unit 112 is operated by the amplified half-wave alternating current power having the negative polarity (-)to generate anions. The cation generating unit 108 and the anion generating unit 112 are separated from each other by a designated distance, thereby preventing the reaction between the cations generated by the cation generating unit 108 and the anions generated by the anion generating unit 112 and preventing the extermination of the cations and the anions due to the reaction thereof. That is, it is possible to remarkably increase the number of the generated cations and anions. A controller 114 controls amplification factors of the first and second amplifying devices 106 and 110, thereby controlling ion-generating capacities of the cation generating unit 108 and the anion generating unit 112.

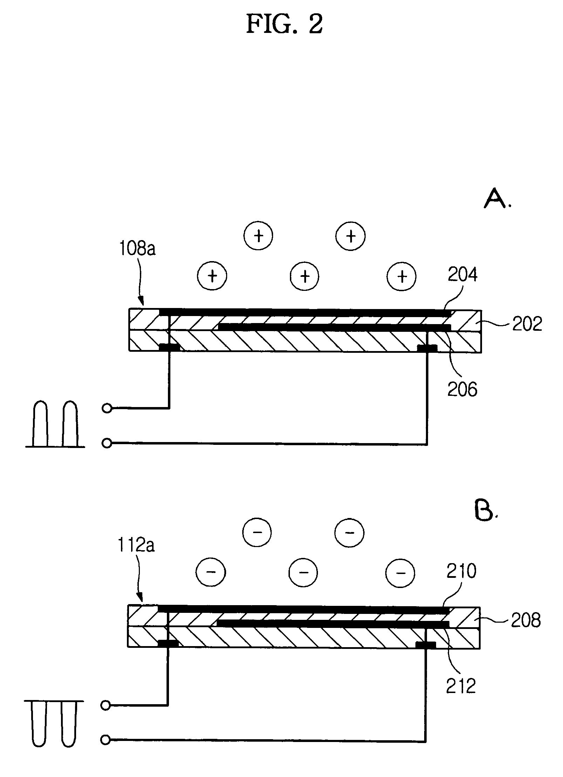

FIG. 2A is a schematic view illustrating a cation generating unit of the ion generator of FIG. 1 in accordance with an exemplary embodiment of the present invention. As shown in FIG. 2A, a cation generating unit 108a includes a first ceramic plate 202, and a first discharge electrode 204 and a first induction electrode 206, which are separated from each other and embedded in the first ceramic plate 202. The half-wave alternating current power having the positive polarity (+) amplified by the first amplifying device 106 is supplied to the first discharge electrode 204 and the first induction electrode 206. When the above high voltage having the positive polarity (+) is applied to the first discharge electrode 204 and the first induction electrode 206, the first ceramic plate 202 generates cations by plasma discharge.

FIG. 2B is a schematic view illustrating an anion generating unit of the ion generator of FIG. 1 in accordance with an exemplary embodiment of the present invention. As shown in FIG. 2B, an anion generating unit 112a includes a second ceramic plate 208, and a second discharge electrode 210 and a second induction electrode 212, which are separated from each other and embedded in the second ceramic plate 208. The half-wave alternating current power having the negative polarity (-) amplified by the second amplifying device 110 is supplied to the second discharge electrode 210 and the second induction electrode 212. When the above high voltage having the negative polarity (-) is applied to the second discharge electrode 210 and the second induction electrode 212, the second ceramic plate 208 generates anions by plasma discharge.

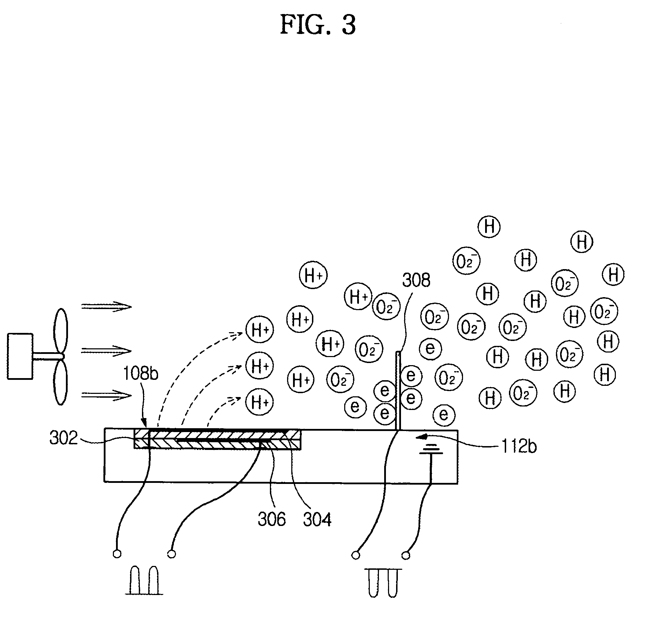

FIG. 3 is a schematic view illustrating a cation generating unit and an anion generating unit of the ion generator of FIG. 1 in accordance with another exemplary embodiment of the present invention. As shown in FIG. 3, a cation generating unit 108b includes a first ceramic plate 302, and a first discharge electrode 304 and a first induction electrode 306, which are separated from each other and embedded in the first ceramic plate 302. The half-wave alternating current power having the positive polarity (+) amplified by the first amplifying device 106 is supplied to the first discharge electrode 304 and the first induction electrode 306. When the above high voltage having the positive polarity (+) is applied to the first discharge electrode 304 and the first induction electrode 306, the first ceramic plate 302 ionizes moisture (H₂O) in the air by plasma discharge, thereby generating hydrogen ions (H⁺).

An anion generating unit 112b includes a needle-shaped electrode 308 which is supplied with the half-wave alternating current power having the negative polarity (-) amplified by the second amplifying device 110.

When the above high voltage having the negative polarity (-) is applied to an area between the needle-shaped electrode 308 and a ground electrode, anions are accumulated around the needle-shaped electrode 308 by plasma discharge, and the needle-shaped electrode 308 generates a large quantity of electrons on its surface. The large quantity of the electrons exhausted to the air are captured by oxygen molecules (O₂) in the air, thereby producing superoxide anions (O₂⁻). Accordingly, the above high voltage having the negative polarity (-) is applied to the needle-shaped electrode 308, and the needle-shaped electrode 308 generates electrons and superoxide anions (O₂⁻).

When the electrons are exhausted from the needle-shaped electrode 308, the electrons react with the hydrogen ions (H⁺), which are generated from the ceramic plate 302 and pass through the circumference of the needle-shaped electrode 308, thereby producing hydrogen atoms (H, or active hydrogen). As described above, the hydrogen ions generated from the ceramic plate 302 react with the electrons generated from the needle-shaped electrons 308 to produce the hydrogen atoms (H). Accordingly, substances, which are finally exhausted from the ion generator of the present invention, are hydrogen atoms (H) and superoxide anions (O₂⁻).

As apparent from the above description, the present invention provides an ion generator, which generates cations and anions respectively through cation and anion generating units independently installed so as to prevent the extermination of the cations and anions due to the reaction thereof just after the generation of the cations and anions, thereby being capable of generating a large quantity of the cations and anions.

Although exemplary embodiments of the invention has been shown and described, it would be appreciated by those skilled in the art that changes may be made in these embodiments without departing from the principles and spirit of the invention, the scope of which is defined in the claims and their equivalents.

## Claims

1. An ion generator comprising:
a power supply device which supplies alternating current power;
a rectifying device which rectifies the alternating current power, supplied from the power supply device, into a half-wave alternating current power having a positive polarity and a half-wave alternating current power having a negative polarity;
a first ion generating unit which generates cations in response to the half-wave alternating current power having the positive polarity received from the rectifying device; and
a second ion generating unit which generates anions in response to the half-wave alternating current power having the negative polarity received from the rectifying device.

2. The ion generator according to claim 1, wherein the first ion generating unit and the second ion generating unit are independently installed such that they are separated from each other by a designated distance.

3. The ion generator according to claim 2, wherein:
the first ion generating unit comprises a first ceramic plate, a first discharge electrode and a first induction electrode, the first discharge electrode and the first induction electrode are separated from each other and embedded in the first ceramic plate, and the half-wave alternating current power having the positive polarity is supplied to the first discharge electrode and the first induction electrode; and
the second ion generating unit comprises a second ceramic plate, a second discharge electrode and a second induction electrode, the second discharge electrode and the second induction electrode are separated from each other and embedded in the second ceramic plate, and the half-wave alternating current power having the negative polarity is supplied to the second discharge electrode and the second induction electrode.

4. The ion generator according to claim 2, wherein:
the first ion generating unit comprises a first ceramic plate, a first discharge electrode and a first induction electrode, the first discharge electrode and the first induction electrode are separated from each other and embedded in the first ceramic plate, and the half-wave alternating current power having the positive polarity is supplied to the first discharge electrode and the first induction electrode; and
the second ion generating unit comprises a needle-shaped electrode, and the half-wave alternating current power having the negative polarity is supplied to the needle-shaped electrode.

5. The ion generator according to claim 1, further comprising:
a first amplifying device which amplifies the half-wave alternating current power having the positive polarity and supplies the amplified half-wave alternating current power having the positive polarity to the first ion generating unit;
a second amplifying device which amplifies the half-wave alternating current power having the negative polarity and supplies the amplified half-wave alternating current power having the negative polarity to the second ion generating unit; and
a controller which controls amplification degrees of the first and second amplifying devices to control ion-generating capacities of the first and second ion generating units.

6. An ion generator comprising:
a power supply device which supplies alternating current power;
a rectifying device which rectifies the alternating current power, supplied from the power supply device, into a half-wave alternating current power having a positive polarity and a half-wave alternating current power having a negative polarity;
a first amplifying device which amplifies the half-wave alternating current power having the positive polarity amplified by the amplifying device;
a first ion generating unit which generates cations in response to receiving the half-wave alternating current power having the positive polarity amplified by the amplifying device;
a second amplifying device which amplifies the half-wave alternating current power having the negative polarity amplified by the amplifying device;
a second ion generating unit which generates anions in response to receiving the half-wave alternating current power having the negative polarity amplified by the amplifying device; and
a controller which controls amplification factors of the first and second amplifying devices to control ion-generating capacities of the first and second ion generating units.
